# EUROPEAN PATENT APPLICATION

(11) **EP 4 609 830 A1**
(43) Date of publication of application: **03.09.2025**
(21) Application number: 23881930.4
(22) Date of filing: 26.10.2023
(51) Int. Cl.: A61F 2/24

(54) **MEDICAL DEVICE**

(30) Priority: 27.10.2022 CN 202211327911; 27.10.2022 CN 202211328166
(71) Applicant: United Innomed (Shanghai) Limited, Shanghai 201315 (CN)
(72) Inventor: WANG, Li, Shanghai 201315 (CN)
(74) Representative: Winter, Brandl - Partnerschaft mbB
(86) International application number: PCT/CN2023/126911
(87) International publication number: WO 2024/088355

(57) **Abstract**

A medical device (20) for repairing a valve of a patient. The valve comprises a plurality of leaflets. The medical device (20) comprises pads (21) configured to be located between the plurality of leaflets of the valve so as to periodically open and close the valve by cooperating with the plurality of leaflets. By means of the pads (21) located between the plurality of leaflets, the medical device (20) can improve the closure condition of the valve, so that the valve can be closed appropriately, thereby effectively preventing or reducing the backflow of blood. In addition, the medical device (20) causes less interference to the blood flow, such that to the occurrence of stenosis can be reduced. Moreover, the medical device (20) affects the movement of leaflets less, thereby having less adverse effects on the structure and function of the leaflets.

## Description

### TECHNICAL FIELD

The present application relates to the field of medical apparatus and instruments technologies, and in particular, to a medical device for repairing a heart valve of a patient.

### BACKGROUND

A heart valve refers to a valve located between an atrium and a ventricle or a valve located between the ventricle and an artery. The valve plays a critical role in the heart' ceaseless blood circulation. After blood flows through, the valve closes to prevent blood regurgitation.

Various structural factors may affect proper closure of the heart valve, and thereby inducing the blood regurgitation. Taking mitral valve as an example, if the mitral valve fails to close properly, the blood may flow from the left ventricle to the left atrium through the mitral valve during systole, and thereby impairing a patient's health. A repair device for repairing the heart valve provided by related technologies has a structure similar to a clip. This medical device improves the condition of valve closure insufficiency by clipping onto a pair of leaflets of the valve.

However, such repair device has may defects. For instance, an effective area for the blood to flow through the valve is reduced due to such repair device, and so that another issue may be caused-stenosis. Additionally, as this repair device clips the pair of leaflets together, the pair of leaflets are prevented from naturally coaptating and separating during a cardiac cycle. As time goes on, this may cause problems to the structure and function of the pair of leaflets, then their associated tissues, and even the entire valve.

### SUMMARY

In view of the above, the present application provides a medical device.

The medical device provided by the present application is used for repairing a patient's heart valve. This medical device includes a pad configured to be capable of being positioned among a plurality of leaflets of the valve to make the valve periodically open and close by cooperating with the leaflets.

By the pad located among the plurality of leaflets, the medical device provided by the present application is able to improve the closure condition of the valve, so that the valve is properly closed, and thus the regurgitation of blood is effectively prevented or reduced. Furthermore, the medical device provided by the present application has less interference to blood flow, and therefore is able to reduce the possibility of stenosis. In addition, the medical device provided by the present application has a minimal influence on the movement of the leaflets, so that fewer adverse effects on the structure and function of the leaflets are caused.

In a possible implementation, the plurality of leaflets includes a first leaflet and a second leaflet, the pad has a first coaptation surface, the first coaptation surface is configured to face the first leaflet and periodically coaptate and separate from the first leaflet as the first leaflet or the second leaflet moves, and the first coaptation surface is configure to be concave in a direction away from the first leaflet.

The coaptation surface is constructed to be concave in a direction away from the leaflet it faces, so that the coaptation surface is able to better match with this leaflet, and thereby enabling this coaptation surface to coaptate the leaflet more effectively.

In a possible implementation, the valve allows blood to flow from a first chamber to a second chamber and prevent flowing in a reverse direction, and the first coaptation surface is configured to gradually extend toward a side where the first leaflet is located in a direction trending towards the first chamber.

In some cases, there may still be a small amount of blood flow back from the second chamber to the first chamber. The coaptation surface is constructed to be concave in a direction away from the leaflet it faces and gradually extend toward the side where the leaflet is located while trending towards the first chamber, so that the flow direction of blood from the second chamber to the first chamber is changed. It guides the blood flow to one side to avoid the direct impact of the blood flow on the first chamber, and thereby reducing the adverse effects of regurgitation on the patient's cardiac function.

In a possible implementation, the first coaptation surface has a first edge portion and a second edge portion respectively located at two opposite ends of the first coaptation surface, the first edge portion is configured to be closer to the first chamber relative to the second edge portion, and an included angle formed between a tangent plane of the first edge portion and a tangent plane of the second edge portion ranges from 10° to 45°.

The greater the included angle between the tangent plane of the first edge portion and the tangent plane of the second edge portion, the more significant the change in the direction of blood flow from the second chamber to the first chamber caused by the coaptation surface. The included angle between the two is set at this range, so that the blood flow from the second chamber to the first chamber is effectively guided to one side while ensuring good coaptation between the leaflet and the coaptation surface.

In a possible implementation, the pad also has a top surface, and the top surface is configured to face the first chamber and protrude toward the first chamber.

To better match with the plurality of leaflets, a thickness of the pad gradually increases while trending towards the first chamber, which may lead to an increased likelihood of blood accumulation at the top of the pad. The top surface of the pad is constructed to protrude toward the first chamber, so that the accumulation of blood at the top of the pad is effectively avoided or reduced.

In a possible implementation, wherein the pad has a plurality of coaptation surfaces. The plurality of coaptation surfaces are configured to face the plurality of leaflets respectively, and as the plurality of leaflets move, each coaptation surface periodically coaptates and separates from the corresponding leaflet it faces.

In this implementation, the physiological cyclic movement of each leaflet will not be interfered with or will be minimally interfered with, so that the medical device will cause fewer problems to the structure and function of the leaflets. Moreover, the valve is able to periodically open and close through its own movement, and effective closure of the leaflets is increased through the coaptation of the pad and the leaflet during systole. Therefore, it not only able to allow the blood to automatically flow from the first chamber to the second chamber during diastole, but also reverse flow during systole caused by poor coaptation of the original two leaflets is effectively prevented.

In a possible implementation, the top surface is configured to protrude from a central portion of the top surface and thus extend gradually toward the second chamber while trending towards a side where the leaflet is located.

This construction of the top surface is capable of effectively avoiding or reducing the accumulation of blood on the top of the pad. In addition, this construction of the top surface is also capable of reducing the obstruction of the pad to the blood flow from the first chamber to the second chamber, so that the blood flow is better guided into the second chamber from the first chamber.

In a possible implementation, an included angle formed between a tangent plane of the central portion of the top surface and a tangent plane of an edge portion of the top surface ranges from 10° to 45°, and the edge portion of the top surface is close to the leaflet.

In this way, the top surface is capable of better guiding the blood flow into the second chamber from the first chamber, so that the obstruction of the pad to the blood flow from the first chamber to the second chamber is reduced.

In a possible implementation, the medical device also includes a support member, the support member is configured to be located in the first chamber and be connected to the pad such that positioning the pad between the plurality of leaflets.

By the support member placed in the first chamber, the pad is able to be reliably positioned between the plurality of leaflets.

In a possible implementation, the support member has a main body portion and a pair of connecting portions, and the pair of connecting portions are configured to extend to two opposite sides of the pad along a width direction of the pad respectively and be connected to the pad.

When the valve is open, most of the blood will flow along the height direction from the two sides in the thickness direction of the pad, and finally flow into the second chamber. Since most of the blood flows past the two sides in the thickness direction of the pad, this construction of the support member is able to significantly reduce the obstruction of the portion the support member connected to the pad to blood flow, and thereby reducing the adverse effects of the medical device on the patient's normal physiological functions.

In a possible implementation, the pad includes an expandable mesh frame and a cover covering an outer side of the mesh frame, and the cover is disposed at a top end of the pad without covering any of the plurality of coaptation surfaces.

A shape and size of the pad generally depend on the mesh frame. During delivery, the mesh frame may be folded to reduce an overall size of the pad to make the delivery process be easier. After the pad is delivered to a target position, the mesh frame may be expanded to make the pad have an appropriate size and shape. Moreover, this construction with a mesh frame allows a physician to adaptively adjust the shape and size of the pad according to the differences in the valves of different patients, so that the pad is able to better match with the patient's valve. The cover covered outside the mesh frame is able to provide a smooth surface for the pad, which on one hand is able to avoid or reduce tissue overgrowth and blood adhesion on the pad, and on the other hand enable the pad to tightly coaptate the leaflets. The cover is merely provided on the top end of the pad without covering the coaptation surface, so that facilitating faster epithelialization of the coaptation surface, and thus reducing the impact on the leaflet tissue when the leaflet comes into contact with the coaptation surface.

In a possible implementation, the pad has a second coaptation surface, the second coaptation surface is configured to face the second leaflet and periodically coaptate and separate from the second leaflet as the second leaflet moves, and the second coaptation surface is configured to be concave in a direction away from the second leaflet.

In a possible implementation, the valve allows blood to flow from a first chamber into a second chamber and prevents flowing in a reverse direction, and the second coaptation surface is configured to extend gradually toward a side where the second leaflet is located while trending towards the first chamber.

In a possible implementation, the second coaptation surface has a first edge portion and a second edge portion located at two opposite ends of the second coaptation surface, the first edge portion is configured to be closer to the first chamber relative to the second edge portion, and an included angle formed between a tangent plane of the first edge portion and a tangent plane of the second edge portion ranges from greater than or equal to 10° to less than or equal to 45°.

In a possible implementation, the pad is configured to follow movement of the second leaflet, and the first coaptation surface is configured to periodically coaptate and separate from the first leaflet as the pad follows the movement of the second leaflet.

In this implementation, the physiological cyclic movement of each leaflet will not be interfered with or will be minimally interfered with, so that the medical device will cause fewer problems to the structure and function of the leaflets. Moreover, the valve is able to periodically open and close through its own movement, and effective closure of the leaflets is increased through the coaptation of the pad and the leaflet during systole. Therefore, it not only able to allow the blood to automatically flow from the first chamber to the second chamber during diastole, but also reverse flow during systole caused by poor coaptation of the original two leaflets is effectively prevented.

In a possible implementation, the top surface is configured to extend gradually toward a side where the first leaflet is located while trending towards the second chamber.

This construction of the top surface is capable of effectively avoiding or reducing the accumulation of blood on the top of the pad. In addition, this construction of the top surface is also capable of reducing the obstruction of the pad to the blood flow from the first chamber to the second chamber, so that the blood flow is better guided into the second chamber from the first chamber.

In a possible implementation, the top surface has a third edge portion and a fourth edge portion located at two opposite ends of the top surface, the fourth edge portion is configured to be closer to the second chamber relative to the third edge portion, and an included angle formed between a tangent plane of the third edge portion and a tangent plane of the fourth edge portion ranges from greater than or equal to 10° to less than or equal to 45°.

In this way, the top surface is capable of better guiding the blood flow into the second chamber from the first chamber, so that the obstruction of the pad to the blood flow from the first chamber to the second chamber is reduced.

In a possible implementation, the pad also has a second coaptation surface, the second coaptation surface is configured to face the second leaflet and be concave in a direction away from the second leaflet, and remain coaptating the second leaflet as the pad follows the movement of the second leaflet.

The second coaptation surface faces the second leaflet and is concave in a direction away from the second leaflet. This construction allows the second coaptation surface to better match and fit with to the second leaflet, and thereby effectively avoiding or reducing the accumulation of blood between the second coaptation surface and the second leaflet.

In a possible implementation, the pad is configured to be attached to the second leaflet.

The pad is attached to the second leaflet, so that it is able to move with the second leaflet.

In a possible implementation, the valve allows blood to flow from a first chamber into a second chamber and prevent flowing in a reverse direction, the medical device further includes a support member, the support member is configured to be located in a first chamber and position the pad between the first leaflet and the second leaflet, and the pad is connected to the support member in a manner that allows the pad to follow the movement of the second leaflet.

Through the support member placed in the first chamber, the pad is reliably positioned between the first leaflet and the second leaflet. The pad is connected to the support member in a manner that allows it to follow the movement of the second leaflet. This ensures that as the second leaflet moves, the pad moves with it, and thereby enabling the first coaptation surface to periodically coaptate and separate from the first leaflet.

In a possible implementation, a thickness of the pad gradually increases while trending towards a first chamber.

In a possible implementation, the valve is a mitral valve, and a quantity of the plurality of leaflets is two; or, the valve is a tricuspid valve, and a quantity of the plurality of leaflets is three.

### BRIEF DESCRIPTION OF THE DRAWINGS

In order to more clearly illustrate technical solutions of embodiments of the present application, the drawings to be used in the embodiments will be introduced briefly below.

It should be understood that, the drawings below merely show some embodiments of the present application, but not all the embodiments.

It should be understood that, the same or similar reference numerals are used in the drawings for representing the same or similar elements.

It should be understood that, the drawings are merely illustrative, and dimensions and proportions of the elements in the drawings are not necessarily accurate.
FIG. 1 is a schematic structural diagram of a medical device according to an embodiment of the present application.
FIG. 2 is a schematic structural diagram of a medical device according to another embodiment of the present application.
FIG. 3A and FIG. 3B are schematic structural diagrams of a pad of the medical device of FIG. 2.
FIG. 4 is an axonometric schematic structural diagram of the medical device in FIG. 2.
FIG. 5 is a schematic structural diagram of a pad of a medical device according to another embodiment of the present application.
FIG. 6 is a schematic structural diagram of a medical device according to yet another embodiment of the present application.
FIG. 7 is a schematic structural diagram of a medical device according to still another embodiment of the present application.
FIG. 8A and FIG. 8B are schematic structural diagrams of a pad of the medical device of FIG. 7.
FIG. 9 is a schematic structural diagram of a medical device according to further another embodiment of the present application.
FIG. 10 is a schematic structural diagram of a medical device according to yet a further embodiment of the present application.

### DETAILED DESCRIPTION OF THE EMBODIMENTS

To make a person skilled in the art better understand technical solutions of the present application, the technical solutions of implementations of the present application will be described below with reference to the drawings. Apparently, the described implementations are merely some implementations of the present application, rather than all the implementations.

It is considered that the repair device for repairing the valve that has a structure similar to a clip has many defects as mentioned above, the present application provides a novel medical device for repairing a patient's valve. The medical device provided by the present application includes a pad configured to be located among a plurality of leaflets of the valve, to make the valve periodically open and close by cooperating with the plurality of leaflets.

By the pad located among the plurality of leaflets, the medical device provided by the present application is able to improve the closure condition of the valve, so that the valve is properly closed, and thus the regurgitation of blood is effectively prevented or reduced. Furthermore, the medical device provided by the present application has less interference to blood flow, and therefore is able to reduce the possibility of stenosis. In addition, the medical device provided by the present application has a minimal influence on the movement of the leaflets, so that fewer adverse effects on the structure and function of the leaflets are caused.

The valve involved in the present application may be heart valves. In particular, the valve may be a mitral valve or tricuspid valve. When the valve is a mitral valve, the plurality of leaflets involved in the present application may be the two leaflets of the mitral valve. When the valve is a tricuspid valve, the plurality of leaflets may be the three leaflets of the tricuspid valve. It should be understood that, the medical device provided by the present application is not limited to application in mitral and tricuspid valves, but may also be applied to other valves.

The medical device provided by the present application will be described below with reference to the drawings.

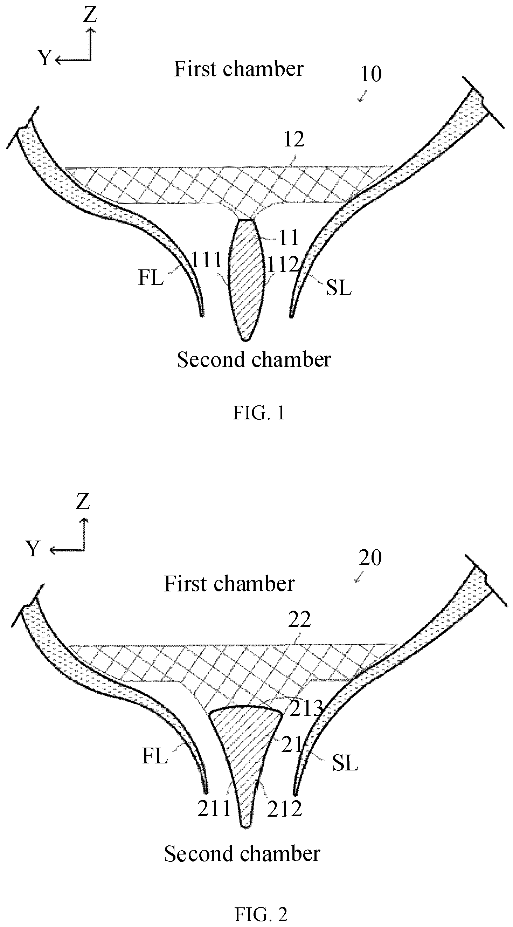

FIG. 1 is a schematic structural diagram of a medical device 10 according to an embodiment of the present application, and FIG. 1 illustrates a state that the medical device 10 is placed in a heart valve of a patient.

In FIG. 1 (and other figures of the present application), the heart valve is located between a first chamber and a second chamber of the heart, to allow blood to flow from the first chamber to the second chamber while preventing flowing in a reverse direction. The heart valve includes a pair of leaflets FL, SL matching with each other.

In a normal condition, the leaflets FL, SL are able to naturally coaptate to close the valve and separate to open the valve during a cardiac cycle, so that the blood is allowed to flow from the first chamber to the second chamber while preventing flowing in a reverse direction. However, for a patient with valvular insufficiency, when the valve is required to close, the leaflets FL, SL fail to achieve proper coaptation. For example, a degree of coaptation is significantly reduced, and even there is a gap between the leaflets FL, SL. Therefore, this makes the blood flow from the second chamber back into the first chamber through the valve, and thus resulting regurgitation during systole.

The valve shown in FIG. 1 (as well as in other figures of the present application) may be a mitral valve. Correspondingly, the first chamber is the left atrium, the second chamber is the left ventricle, and one of the leaflets FL, SL is the anterior leaflet while the other is the posterior leaflet. It should be understood that, the medical device provided in the present application is not limited to application in the mitral valve, but may also be applied to other heart valves.

Referring to FIG. 1, the medical device 10 includes a pad 11. When the medical device 10 is implanted in a patient's heart, the pad 11 is located between the leaflet FL and leaflet SL. The pad 11 has a pair of coaptation surfaces 111, 112. When the medical device 10 is implanted in the patient's heart, the pair of coaptation surfaces 111, 112 respectively face the pair of leaflets FL, SL, namely the coaptation surface 111 faces the leaflet FL and the coaptation surface 112 faces the leaflet SL. With the changes of the cardiac cycle, each coaptation surface of the pair of coaptation surfaces 111, 112 periodically coaptates and separates from the leaflet it faces. Specifically, when the valve closes, the coaptation surface 111 coaptates the leaflet FL and the coaptation surface 112 coaptates the leaflet SL to prevent the blood flowing from the second chamber back to the first chamber. When the valve opens, the leaflets FL, SL separate from coaptation surfaces 111, 112 respectively to allow the blood to flow from the first chamber into the second chamber. In an example, a coaptation length between each coaptation surface and its corresponding leaflet may range from 6 mm to 12 mm. In an example, the coaptation surfaces 111, 112 may be smooth transition curved surfaces to reduce resistance to the blood flow.

In this implementation, by the pad located among the plurality of leaflets, the medical device provided by the present application is able to improve the closure condition of the valve, so that the valve is properly closed, and thus the regurgitation of blood is effectively prevented or reduced. Furthermore, in this implementation, the medical device has less interference to blood flow, and therefore it is able to reduce the possibility of stenosis. In addition, in this implementation, the medical device has no influence or a minimal influence on the physiological cyclic movement of the leaflets, so that fewer adverse effects on the structure and function of the leaflets are caused. Moreover, the valve is able to be opened and closed periodically by their own movement, and effective closure of the leaflets is increased through the coaptation of the pad and the leaflet during systole. Therefore, it not only able to allow the blood to automatically flow from the first chamber to the second chamber during diastole, but also reverse flow during systole caused by poor coaptation of the original two leaflets is effectively prevented.

Referring again to FIG. 1, the medical device 10 may further include a support member 12. When the medical device 10 is implanted in a patient's heart, the support member 12 is located in the first chamber and is connected to the pad 11, so that the pad 11 is positioned between the pair of leaflets SL, FL. By placing the support member 12 in the first chamber, the pad 11 is able to be reliably positioned between the pair of leaflets FL, SL. In an example, the pad 11 may be rigidly connected to the support member 12 to consistently maintain the pad 11 in a proper position, avoiding changes in the position of the pad 11 due to the influence of blood flow or the leaflets FL, SL. If the pad 11 cannot be maintained in a proper position, it cannot be ensured that the pair of leaflets FL, SL will achieve good coaptation with the pair of coaptation surfaces 111, 112 each time the valve closes.

The support member may be implemented in various ways, which are not specifically limited in the present application.

As an example, referring again to FIG. 1, the support member 12 may be annular in shape. When the medical device 10 is implanted in a patient's heart, the support member 12 may be placed at the annulus of the valve of the heart. In some embodiments, the support member 12 may have a mesh frame structure. During delivery, the support member 12 may firstly be folded to reduce its volume, to make the delivery process be easy. Once the support member 12 is delivered to a target position, it may be unfolded again.

It should be understood that, the implementation of the support member is not limited to the above, as long as it is able to position the pad between the pair of leaflets of the valve. For example, in some embodiments, the support member may also be semi-annular or partially annular. In some embodiments, the support member may be made of a flexible material, so that it is able to deform in accordance with systole and diastole of the first chamber, and thereby reducing adverse effects on physiological functions of the heart.

FIG. 2 is a schematic structural diagram of a medical device 20 according to another embodiment of the present application, showing a state that the medical device 20 is placed at a heart valve of a patient.

Referring to FIG. 2, the medical device 20 includes a pad 21. In some embodiments, the pad 21 may be configured such that its thickness gradually increases toward a first chamber, thereby better matching with shapes of a pair of leaflets FL, SL, and thus the closure of the valve is more effectively achieved.

When the medical device 20 is implanted in the patient's heart, the pad 21 is located between the pair of leaflets FL, SL. The pad 21 has a pair of coaptation surfaces 211, 212. When the medical device 20 is implanted in the patient's heart, the pair of coaptation surfaces 211, 212 respectively face the pair of leaflets FL, SL. As the pair of leaflets FL, SL move, each coaptation surface periodically coaptates and separates from the leaflet it faces.

The pair of coaptation surfaces 211, 212 may both be inwardly concave surfaces. Specifically, when the medical device 20 is implanted in the patient's heart, the coaptation surface 211 may be recessed in a direction away from the leaflet it faces (that is, the leaflet FL), and the coaptation surface 212 may be recessed in a direction away from the leaflet it faces (that is, the leaflet SL).

In this way, the pair of coaptation surfaces are able to better match with the leaflets they face respectively, so that the pair of coaptation surfaces better coaptate the leaflets they face respectively.

As an example, referring again to FIG. 2, when the medical device 20 is implanted in the patient's heart, the coaptation surface 211 may be constructed to gradually extend toward a side where the leaflet FL it faces is located (that is, the left side in FIG. 2) in a direction trending towards the first chamber. Similarly, the coaptation surface 212 may be constructed to gradually extend toward a side where the leaflet SL it faces is located (that is., the right side in FIG. 2) in a direction trending towards the first chamber.

In some cases, there may still be a small amount of blood flowing from the second chamber back into the first chamber. By constructing the pair of coaptation surfaces to be concave in directions away from the leaflets they face respectively, and to gradually extend toward the sides where the leaflets they face are located in the direction trending towards the first chamber respectively, so that the flow direction of blood regurgitating from the second chamber to the first chamber is able to be changed, thereby the blood flow being guided to one side to prevent it from directly impacting the first chamber, and thus the direct "impact" of the regurgitation on the patient's atrium and pulmonary veins is reduced to reduce adverse effects on the cardiac function.

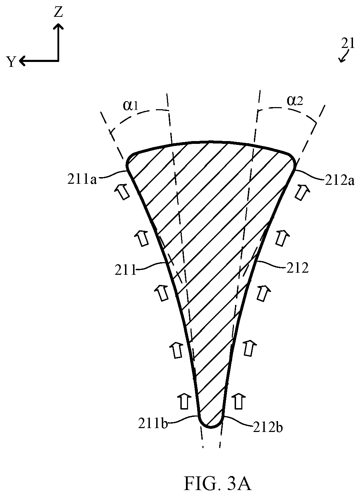

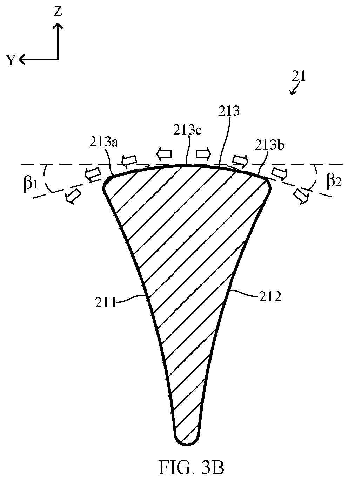

FIG. 3Ais a schematic structural diagram of the pad 21. In FIG. 3A, thick arrows are used for schematically indicating the changing trend of the flow direction of the blood flow that flows from the second chamber to the first chamber under the guidance of the pair of coaptation surfaces 211, 212.

Referring to FIG. 2 and FIG. 3A, the coaptation surface 211 has a first edge portion 211a and a second edge portion 211b located at two opposite ends thereof, respectively. When the medical device 20 is implanted in the patient's heart, the first edge portion 211a is closer to the first chamber than to the second edge portion 211b. Included angles between tangent planes at positions on the coaptation surface 211 and a tangent plane of the second edge portion 211b gradually increase as the positions on the coaptation surface 211 trends towards the first edge portion 211a. An included angle (acute angle) α1 formed between a tangent plane of the first edge portion 211a and the tangent plane of the second edge portion 211b may range from greater than or equal to 10° to less than or equal to 45°. Preferably, the range of α1 may be from greater than or equal to 15° to less than or equal to 30°.

The coaptation surface 212 has a first edge portion 212a and a second edge portion 212b located at two opposite ends thereof, respectively. When the medical device 20 is implanted in the patient's heart, the first edge portion 212a is closer to the first chamber than to the second edge portion 212b. Included angles between tangent planes at positions on the coaptation surface 212 and a tangent plane of the second edge portion 212b gradually increase as the positions on the coaptation surface 212 approach the first edge portion 212a. An included angle (acute angle) α2 formed between a tangent plane of the first edge portion 212a and the tangent plane of the second edge portion 212b ranges from greater than 45° to less than 90°. The included angle α1 and the included angle α2 may be equal or unequal, which is not specifically limited in the present application.

The greater the included angle between the tangent plane of the first edge portion and the tangent plane of the second edge portion, the greater the change in the flow direction of blood flow that flows from the second chamber to the first chamber caused by the coaptation surface. By setting the included angle between the two within the range of 45° to 90°, the blood flow that flows from the second chamber to the first chamber is effectively guided to one side while ensuring proper coaptation between the leaflets and the coaptation surfaces respectively.

Referring again to FIG. 2, the pad 21 may also include a top surface 213, the top surface 213 and may be outwardly convex surfaces. Specifically, when the medical device 20 is implanted in the patient's heart, the top surface 213 of the pad 21 is located at an end of the pad 21 close to the first chamber, and the top surface 213 faces the first chamber and protrudes toward the first chamber.

To better match with the pair of leaflets of the valve, the thickness of the pad may be constructed to gradually increase towards the first chamber, which may cause the blood to be more easily accumulated at the top of the pad. In the above implementation of the present application, since the pad has a top surface that is convex (in the direction toward the first chamber), so that the accumulation of the blood at the top of the pad is effectively prevented or reduced.

In an example, referring again to FIG. 2, when the medical device 20 is implanted in the patient's heart, the top surface 213 of the pad 21 may be constructed to trend towards the side where each of the pair of leaflets FL, SL is located while gradually extend toward the second chamber from its middle portion.

Specifically, as shown in FIG. 2, the top surface 213 trends towards the side where the leaflet FL is located while (that is, the left side in FIG. 2) gradually extending toward the second chamber (that is, gradually extending downward in FIG. 2) from its middle portion. Meanwhile, the top surface 213 approaches the side where the leaflet SL is located (that is, the right side in FIG. 2) while gradually extending toward the second chamber (that is, gradually extending downward in FIG. 2) from its middle portion. In other words, the top surface 213 has a configuration that is higher in the middle and lower at both ends.

This construction of the top surface is capable of effectively avoiding or reducing the accumulation of blood on the top of the pad. In addition, this construction of the top surface is also capable of reducing the obstruction of the pad to the blood flow from the first chamber to the second chamber, so that the blood flow is better guided into the second chamber from the first chamber.

FIG. 3B is a schematic structural diagram of the pad 21. In FIG. 3B, thick arrows are used for schematically indicating the changing trend of the flow direction of blood flow that flows from the first chamber to the second chamber under the guidance of the top surface 213 of the pad 21.

Referring to FIG. 2 and FIG. 3B, the top surface 213 has edge portions 213a, 213b at two ends thereof in the thickness direction of the pad 21, respectively. When the medical device 10 is implanted in the patient's heart, the edge portion 213a is closer to the leaflet FL (or closer to the coaptation surface 211) than the edge portion 213b, and the edge portion 213b is closer to the leaflet SL (or closer to the coaptation surface 212) than the edge portion 213a.

An included angle between a tangent plane of the middle portion 213c of the top surface 213 and a tangent plane of the edge portion 213a is defined as β1, and an included angle between the tangent plane of the middle portion 213c and a tangent plane of the edge portion 213b is defined as β2. The included angles β1 and β2 may range from greater than or equal to 10° to less than or equal to 45°. In an example, the included angles β1 and β2 may range from greater than or equal to 15° to less than or equal to 30°. In an example, the top surface 213 may be a smooth transition curved surface to reduce resistance to blood flow.

The configuration of the top surface is able to better guide the blood flow that flows from the first chamber to the second chamber, so that obstruction to the blood flow that flows from the first chamber to the second chamber is reduced.

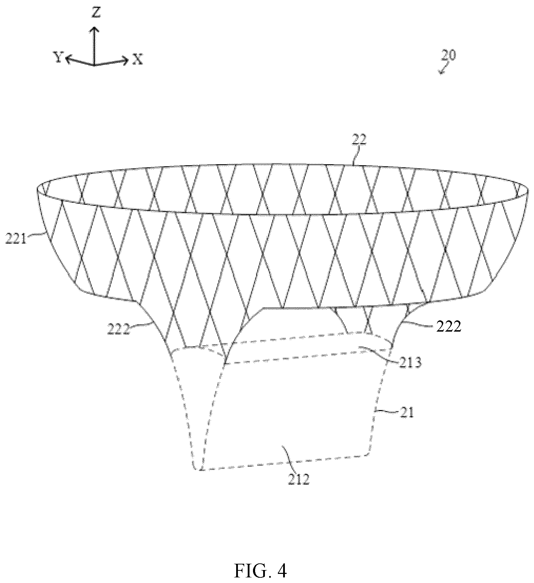

FIG. 4 is an axonometric schematic structural diagram of the medical device 20.

It should be noted that, in the drawings of the present application, an arrow X is used for indicating a width direction of a pad, an arrow Y is used for indicating a thickness direction of the pad, and an arrow Z is used for indicating a height direction of the pad.

As shown in FIG. 4, the support member 22 includes a main body portion 221 and a pair of connecting portions 222. As an example, the main body portion 221 may be annular in shape, and is constructed such that is adapted to be mounted at the valve annulus. Certainly, in other examples, the main body portion 221 may be semi-annular or other shapes. The pair of connecting portions 222 extend respectively to two sides of the pad 21 in the width direction and are connected to the pad 21.

The pair of leaflets are located on two sides of the pad in the thickness direction, and the first chamber and the second chamber are located on two sides of the pad in the height direction. The thickness direction, height direction and width direction of the pad are mutually perpendicular to each other. When the valve opens, most of the blood will flow along the height direction and passes through two sides of the pad in the thickness direction, and eventually flows into the second chamber. Since most of the blood flows through the two sides of the pad in the thickness direction, so that this configuration of the support member is able to reduce the obstruction to the blood flow caused by the portions of the support member connected to the pad to a greater extent, and thus the influence of the medical device on the patient's normal physiological functions is reduced.

It should be noted that, in the various embodiments of the present application described above, any one of the leaflet FL and leaflet SL may be the "first leaflet" mentioned in other sections (for example, the Summary section). Correspondingly, the coaptation surface facing the "first leaflet" may be the "first coaptation surface". The other one of the leaflet FL and leaflet SL may be the "second leaflet" mentioned in other sections (for example, the Summary section). Correspondingly, the coaptation surface facing the "second leaflet" may be the "second coaptation surface".

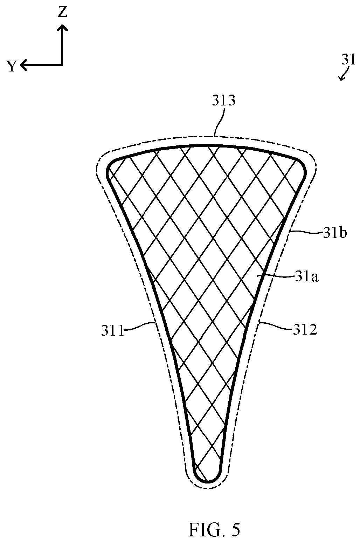

FIG. 5 is a schematic structural diagram of a pad 31 of a medical device according to an embodiment of the present application.

As shown in FIG. 5, the pad 31 includes an expandable mesh frame 31a and a cover 31b covering the mesh frame 31a. The pair of coaptation surfaces 311, 312 and the top surface 313 may be a portion of an outer surface of the cover 31b. In an example, the cover 31b may be made of a biocompatible material and has a smooth outer surface to avoid or reduce the wear of the leaflets or discomfort when matching with the leaflets. In another implementation, the cover 31b may be provided merely on the top end of the pad without covering on the coaptation surface. In this way, endothelialization of the coaptation surface is developed more quickly, so that the influence on the leaflet tissue when the leaflets come into contact with the coaptation surface is reduced.

The shape and size of the pad mainly depend on the mesh frame. During delivery, the mesh frame may be folded to reduce an overall size of the pad, thereby facilitating the delivery process. After the pad is delivered to a target position, the mesh frame may be unfolded to make the pad have an appropriate size and shape. Additionally, this configuration having the mesh frame allows a doctor to adaptively adjust the shape and size of the pad according to differences in different patients' valve, so that the pad is able to better fit with the patient's valves. The cover covering the outer side of the mesh frame provides the pad 31 with a smooth surface, which on one hand helps to prevent or reduce tissue overgrowth or blood adhesion on the pad, and on the other hand enables the pad to coaptate the leaflets tightly.

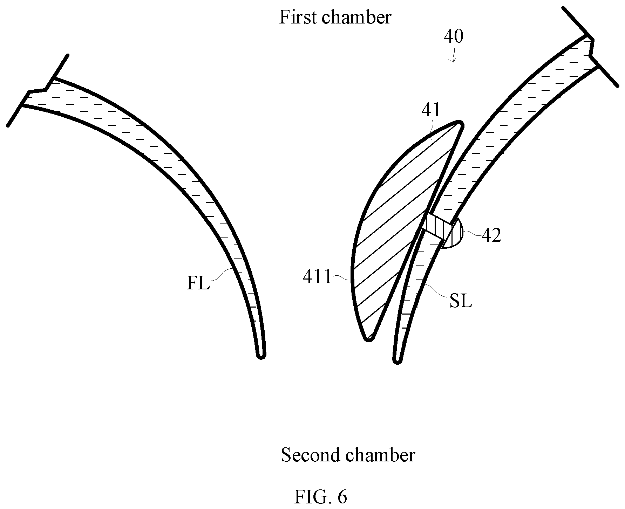

FIG. 6 is a schematic structural diagram of a medical device 40 according to an embodiment of the present application, showing a state that the medical device 40 is placed at a patient's heart valves.

As shown in FIG. 6, the medical device 40 includes a pad 41 having a first coaptation surface 411. When the medical device 40 is implanted in the patient's heart, the pad 41 is located between a first leaflet (FL) and a second leaflet (SL) of the heart valves and moves with the second leaflet SL. The first coaptation surface 411 faces the first leaflet FL and periodically coaptates and separates from the first leaflet FL as the pad 41 moves with the second leaflet SL. In an example, when the first coaptation surface 411 coaptates with the first leaflet FL, a coaptation length between them may range from 6 mm to 12 mm.

Through the pad 41 locating between the pair of leaflets, the medical device 40 is able to improve the closure of the valve, so that the first leaflet FL coaptate the second leaflet SL properly, and thus the regurgitation of the blood is effectively prevented or reduced. Additionally, the medical device 40 has less interferes with blood flow, so that the possibility of stenosis is reduced. Furthermore, the medical device 40 does not affect or has less influence on the motion of the leaflet, so that no or less problems is caused to the function and structure of the leaflets. In other words, by using the medical device 40 provided by the present application, there is no restriction or obstruction on the natural motion of the valve (closing, opening). Meanwhile, it is not associated with the valve's supporting structures, so that there are no adverse effects to be caused. That is, the physiological cyclic motion of each leaflet will not be disturbed or be less disturbed. As a result, the medical device 40 will cause less fewer problems to the structure and function of the leaflets. Moreover, the valve is able to cyclically open and close through their own natural motion, and during systole, sufficient closure of the leaflets is increased through the coaptation between the pad 41 and the leaflet. This not only allows the blood to flow from the first chamber to the second chamber during diastole, but also effectively prevents the regurgitation during systole that caused by the insufficient coaptation between the two leaflets.

The pad 41 may be attached to the second leaflet SL to move with the second leaflet SL. In other words, the pad 41 may be attached to the second leaflet SL in such a way that it follows the motion of the second leaflet SL and becomes a portion of the second leaflet SL, so that the first coaptation surface 411 is able to periodically coaptate and separate from the first leaflet FL with the movement of the second leaflet SL.

As an exemplary implementation, referring again to FIG. 6, the medical device 40 may also include a fixing member 42. In an example, the fixing member 42 may be a pin 42. When the medical device 40 is implanted in the patient's heart, the pin 42 may penetrate through the second leaflet SL to attach the pad 41 to the second leaflet SL, so that the pad 41 is able to move with the second leaflet SL.

The medical device 40 may include only one pin 42, or it may include a plurality of the pins 42. The present application does not specifically limit this. The pin 42 may be integrally formed with the pad 41, or the two may be separate members, which is not specifically limited in the embodiments of the present application.

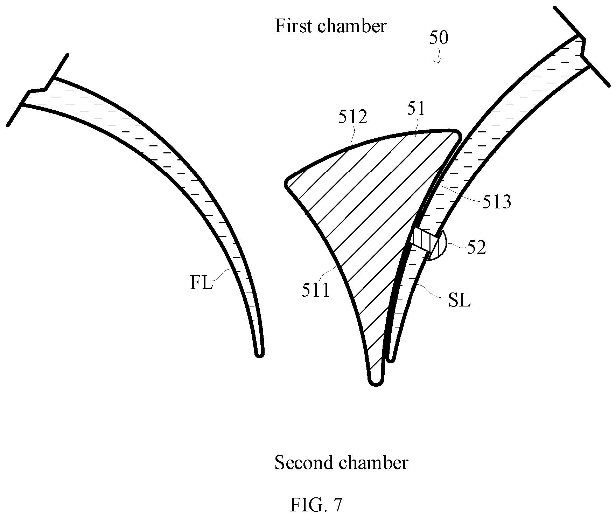

FIG. 7 is a schematic structural diagram of a medical device 50 according to still another embodiment of the present application, showing a state that the medical device 50 is placed at a patient's heart valves.

As shown in FIG. 7, the medical device 50 includes a pad 51 having a first coaptation surface 511. In some embodiments, the pad 51 may be configured such that its thickness gradually increases towards the first chamber, so that the pad 51 better matches with the shapes of the first leaflet FL and the second leaflet SL, and thus the closure of the valve is improved.

When the medical device 50 is implanted in the patient's heart, the pad 51 is located between the first leaflet FL and the second leaflet SL and moves with the second leaflet SL. The first coaptation surface 511 faces the first leaflet FL and periodically coaptates and separates from the first leaflet FL as the pad 51 moves with the second leaflet SL.

The first coaptation surface 511 may be a recessed surface. More specifically, when the medical device 50 is implanted in the patient's heart, the first coaptation surface 511 may be recessed in a direction away from the first leaflet FL. This configuration allows the first coaptation surface 511 to better match with the first leaflet FL, so that better coaptation between the first coaptation surface 511 and the first leaflet FL is achieved.

Referring again to FIG. 7, in a non-limiting example, when the medical device 50 is implanted in the patient's heart, the first coaptation surface 511 gradually extends toward the side where the first leaflet FL is located (that is, the left side in FIG. 7) in the direction trending towards the first chamber.

In some cases, there may still be a small amount of blood flow from the second chamber back into the first chamber. The first coaptation surface 511 (away from the first leaflet FL) is concave and gradually extends toward the side where the first leaflet FL is located in the direction trending towards the first chamber, this configuration enables to increase the resistance of blood flow, so that the regurgitant is reduced, and the flow direction of the blood flow that flows from the second chamber to the first chamber is changed. Therefore, the blood flow is guided to one side, so that the blood flow is prevented from directly impacting the first chamber, and thus the adverse effects on the patient's cardiac function caused by the regurgitant that flows from the second chamber to the first chamber is reduced.

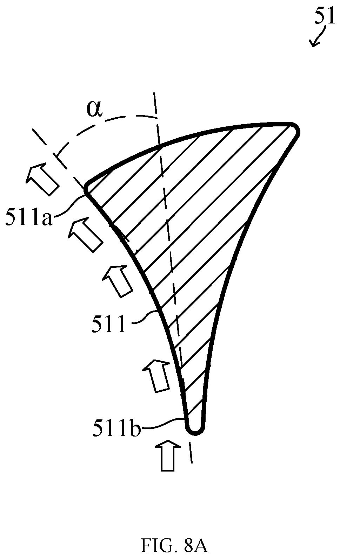

FIG. 8A is a schematic structural diagram of a pad 51. As shown in FIG. 8A, the direction indicated by the thick arrow may represent the flow direction of blood that flows from the second chamber to the first chamber under the guidance of the first coaptation surface 511.

As an exemplary implementation, referring to FIG. 7, FIG. 8A, the first coaptation surface 511 has a first edge portion 511a and a second edge portion 511b located at two opposite ends thereof along the height direction, respectively. When the medical device 50 is implanted in a patient's heart, the first edge portion 511a is closer to the first chamber than the second edge portion 511b.

Included angles between tangent planes at positions on the first coaptation surface 511 and a tangent plane of the second edge portion 511b gradually increase as they approach the first edge portion 511a. An included angle (acute angle) α formed between a tangent plane of the first edge portion 511a and the tangent plane of the second edge portion 511b may range from greater than or equal to 10° to less than or equal to 45°. In an example, the included angle α may range from greater than or equal to 15° to less than or equal to 30°.

The greater the included angle α between the tangent plane of the first edge portion 511a and the tangent plane of the second edge portion 511b, the greater the flow direction of blood flow that flows from the second chamber to the first chamber changed by the first coaptation surface 511. The included angle α of the two is set within this range, so that the blood flow that flows from the second chamber to the first chamber is able to be effectively guided to one side on the basis of ensuring the good coaptation of the first leaflet FL and the first coaptation surface 511.

In an example, referring again to FIG. 7, the pad 51 may also include a top surface 512, and the top surface 512 may be a convex surface. When the medical device 50 is implanted in the patient's heart, the top surface 512 of the pad 51 is located at the end of the pad closer to the first chamber, and the top surface 512 faces the first chamber and protrudes (toward the first chamber).

To better match with the first leaflet FL and the second leaflet SL, the thickness of the pad 51 gradually increases towards the first chamber, which may lead to the accumulation of the blood at the top of the pad 51. Since the pad 51 has a top surface 512 that protrudes (toward the first chamber), so that the accumulation of the blood at the top of the pad 51 is effectively prevented or reduced.

In a non-limiting example, referring again to FIG. 7, when the medical device 50 is implanted in the patient's heart, the top surface 512 of the pad 51 approaches the second chamber while gradually extending toward the side where the first leaflet FL is located (that is, the left side in FIG. 7).

This configuration of the top surface 512 of the pad 51 is able to effectively prevent or reduce the accumulation of the blood at the top of the pad 51. Additionally, this configuration of the top surface 512 of the pad 51 is also able to reduce obstruction and interference to blood flow that flows from the first chamber to the second chamber caused by the pad 51, and thereby better guiding blood flow to flow from the first chamber into the second chamber.

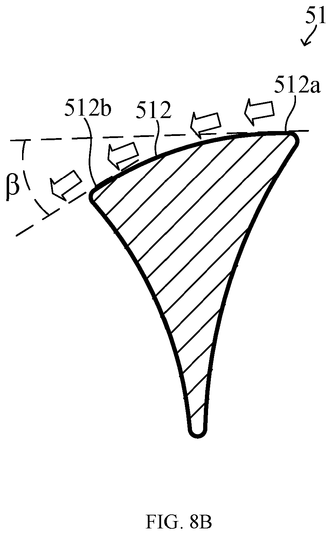

FIG. 8B is a schematic structural diagram of the pad 51. As shown in FIG. 8B, the direction indicated by the thick arrow may represent the flow direction of blood flow that flows from the first chamber to the second chamber under the guidance of the top surface 512 of the pad 51.

In an example, referring to FIGS. 7 and 8B, the top surface 512 of the pad 51 includes a third edge portion 512a and a fourth edge portion 512b located at two opposite ends thereof in the thickness direction, respectively. When the medical device 50 is implanted in the patient' s heart, the fourth edge portion 512b is closer to the second chamber than the third edge portion 512a.

Included angles between tangent planes of the top surface 512 and a tangent plane of the third edge portion 512a gradually increase as they approach the fourth edge portion 512b. An included angle β formed between the tangent plane of the third edge portion 512a and a tangent plane of the fourth edge portion 512b may range from greater than or equal to 10° to less than or equal to 45°. In an example, the included angle β may range from greater than or equal to 15° to less than or equal to 30°.

In this manner, the top surface 512 of the pad 51 is able to better guide the blood to flow from the first chamber to the second chamber, so that the obstruction caused by the pad 51 to the blood flow is reduced.

In a specific example, referring again to FIG. 7, the pad 51 may also include a second coaptation surface 513. The second coaptation surface 513 may be a recessed surface. More specifically, when the medical device 50 is implanted in the patient' s heart, the second coaptation surface 513 faces the second leaflet SL and is recessed away from the second leaflet SL. Additionally, when the medical device 50 is implanted, as the pad 51 moves with the second leaflet SL, the second coaptation surface 513 coaptates the second leaflet SL.

This configuration enables the second coaptation surface 513 of the pad 51 to better match with and fit with the second leaflet SL, so that the accumulation of the blood between the second coaptation surface 513 and the second leaflet SL is effectively prevented or reduced.

In a non-limiting example, referring again to FIG. 7, the medical device 50 may also include a pin 52. When the medical device 50 is implanted in the patient' s heart, the pin 52 may penetrate through the second leaflet SL to attach the pad 51 to the second leaflet SL to make the pad 51 move with the second leaflet SL.

The medical device 50 may include only one pin 52, or it may include a plurality of the pins 52. The present application does not specifically limit this. The pin 52 may be integrally formed with the pad 51, or the two may be separate members, which is not specifically limited in the embodiments of the present application.

There are various methods for attaching the pad to the second leaflet SL, which are not specifically limited in the present application. One possible implementation is exemplarily described with reference to the accompanying drawings below.

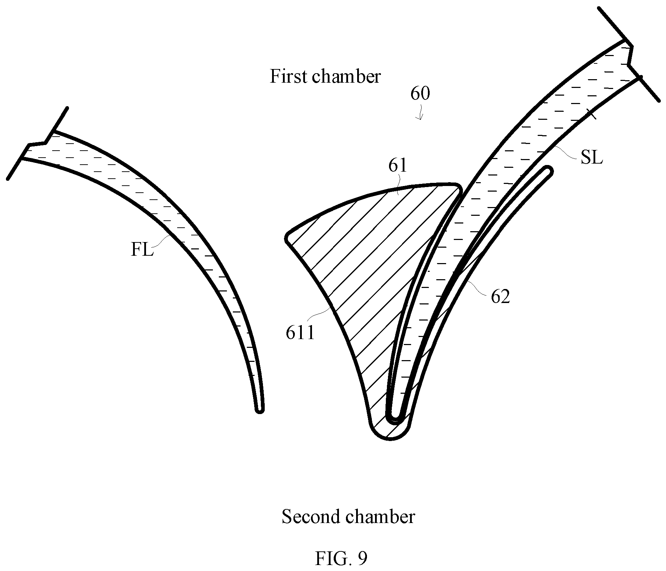

FIG. 9 is a schematic structural diagram of a medical device 60 according to further another embodiment of the present application. FIG. 9 illustrates a state that the medical device 60 is placed at heart valves of a patient. The medical device 60 is substantially the same as the medical device 20, and for the sake of brevity, the similarities will not be described herein again.

Referring to FIG. 9, the medical device 60 includes a pad 61 having a first coaptation surface 611. When the medical device 60 is implanted in the patient's heart, the pad 61 is located between the first leaflet FL and the second leaflet SL and moves with the second leaflet SL. As the second leaflet SL moves, the first coaptation surface 611 periodically coaptates and separates from the first leaflet FL, so that the valve is cyclically closed and opened.

The medical device 60 also includes a clamping member 62, and the clamping member 62 may be integrally formed with the pad 61. Certainly, in other embodiments, the clamping member 62 may also be a member that is separate from the pad 61. When the medical device 60 is implanted in the patient's heart, the clamping member 62 clamps the second leaflet SL to attach the pad 61 to the second leaflet SL, thereby enabling the pad 61 to move with the second leaflet SL.

The present application is not limited to attaching the pad to the second leaflet SL. Other implementations may also be used for positioning the pad between the first leaflet FL and the second leaflet SL and enabling the pad to move with the second leaflet SL.

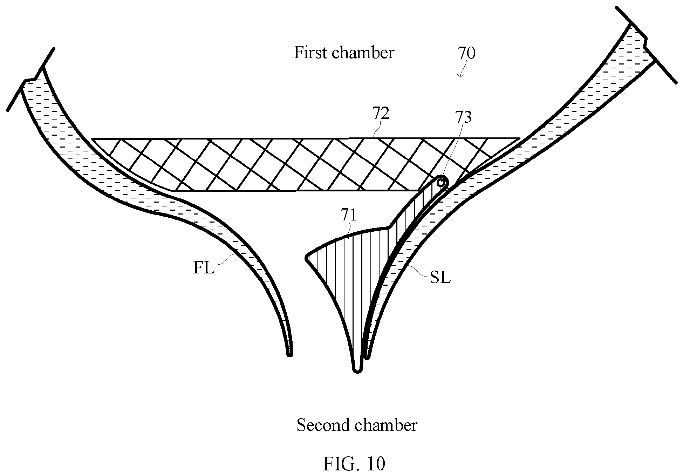

FIG. 10 is a schematic structural diagram of a medical device 70 according to yet another embodiment of the present application. FIG. 10 illustrates a state that the medical device 70 is placed at heart valves of a patient. The medical device 70 is substantially to the same as the medical device 20, and for the sake of brevity, the similarities will not be described herein again.

Referring to FIG. 10, the medical device 70 includes a pad 71 and a support member 72. When the medical device 70 is implanted in the patient's heart, the support member 72 is located in the first chamber, and the pad 71 is connected to the support member 72 in a manner enabling it to move with the second leaflet SL, so that the pad 71 is positioned between the first leaflet FL and the second leaflet SL while following the motion of the second leaflet SL.

In a specific implementation, the pad 71 may be hinged with the support member 72 to make the pad 71 move with the second leaflet SL. In an example, as shown in FIG. 10, the medical device 70 may also include a pivot shaft 73, and the pad 71 is hinged with the support member 72 through the pivot shaft 73. For instance, the pivot shaft 73 may be fixed to the pad 71 and is pivotally connected to the support member 72, and thereby achieving the hinged connection between the pad 71 and the support member 72. Alternatively, the pivot shaft 73 may be fixed to the support member 72 and pivotally connected to the pad 71, and thereby achieving the hinged connection between the pad 71 and the support member 72.

In another implementation, the pad 71 may be connected to the support member via a flexible member (or portion) to enable the pad 71 to move with the second leaflet SL.

By the support member 72 placed in the first chamber, the pad 71 is reliably positioned between the first leaflet FL and the second leaflet SL. The pad 71 is connected to the support member 72 in a manner that the pad 71 is allowed to move with the second leaflet SL, so that as the second leaflet SL moves, the pad 71 moves with the second leaflet SL, and thus the first coaptation surface coaptates and separates from the first leaflet FL periodically.

In an embodiment, the support member 72 may be annular to reduce the influence on the blood flow. Certainly, in other embodiments, the support member 72 maybe semi-annular or partially annular. In an implementation, the support member 72 may be constructed to be relatively soft, or more specifically have flexibility to adaptively deform with the systole and diastole of the first cardiac chamber, so that the adverse effects on the cardiac function are reduced.

There are various implementations of the support member 72, which is not specifically limited in the present application. In a non-limiting example, referring again to FIG. 10, the support member 72 may be in a mesh frame shape that is able to be folded and unfolded. During implanting, the support member 72 may first be folded and then unfolded in the first chamber.

In some embodiments, the support member 72 may be placed at the valve annulus to enable the support member 72 to be reliably fixed.

It should be understood that, although terms such as "first" or "second" may be used in the present application for describing various elements (for example, the first chamber and the second chamber), but these elements are not limited by these terms, and these terms are merely used for distinguishing one element from another.

It should be noted that, the various specific technical features (elements) described in the above detailed embodiments may be combined in any suitable manner without contradiction. To avoid unnecessary repetition, the present application will not be further described in various possible combinations.

It should be understood that, in the embodiments of the present application, "at least one" refers to one or more, and "a plurality of" refers to two or more. The term "and/or" describes the association relationship of associated objects, indicating that three relationships may exist. For example, "A and/or B" may represent a case where A exists alone, both A and B exist, and B exists alone.

It should be understood that, multiple members and/or portions may be provided by a single integrated member or portion. Alternatively, a single integrated member or portion may be divided into separate multiple members and/or portions. The use of "a" or "an" to describe a member or portion is not intended to exclude other members or portions.

The foregoing descriptions are merely specific implementations of the present application, but the protection scope of the present application is not limited thereto. Any person skilled in the art may conceive of modifications or substitutions within the technical scope disclosed in the present application, and such modifications or substitutions should be covered by the protection scope of the present application. Therefore, the protection scope of the present application shall be subject to the scope defined by the claims.

## Claims

1. A medical device for repairing a valve of a patient, comprising a pad, the pad being configured to be positioned among a plurality of leaflets of the valve to make the valve periodically open and close by cooperating with the plurality of leaflets.

2. The medical device according to claim 1, wherein the plurality of leaflets comprises a first leaflet and a second leaflet, the pad has a first coaptation surface, the first coaptation surface is configured to face the first leaflet and periodically coaptate and separate from the first leaflet as the first leaflet or the second leaflet moves, and the first coaptation surface is configure to be concave in a direction away from the first leaflet.

3. The medical device according to claim 2, wherein the valve allows blood to flow from a first chamber to a second chamber and prevent flowing in a reverse direction, and the first coaptation surface is configured to gradually extend toward a side where the first leaflet is located in a direction trending towards the first chamber.

4. The medical device according to claim 3, wherein the first coaptation surface has a first edge portion and a second edge portion respectively located at two opposite ends of the first coaptation surface, the first edge portion is configured to be closer to the first chamber relative to the second edge portion, and an included angle formed between a tangent plane of the first edge portion and a tangent plane of the second edge portion ranges from 10° to 45°.

5. The medical device according to claim 2, wherein the valve allows blood to flow from a first chamber to a second chamber and prevents flowing in a reverse direction, the pad further has a top surface, and the top surface is configured to face the first chamber and protrude toward the first chamber.

6. The medical device according to claim 2, wherein the pad has a plurality of coaptation surfaces which comprise the first coaptation surface, the plurality of coaptation surfaces are configured to face the plurality of leaflets respectively, and as the plurality of leaflets move, each coaptation surface periodically coaptates and separates from the corresponding leaflet it faces.

7. The medical device according to claim 6, wherein the valve allows blood to flow from a first chamber into a second chamber and prevents flowing in a reverse direction, the pad also has an top surface, the top surface is configured to face the first chamber and protrude toward the first chamber, and the top surface is configured to extend gradually toward the second chamber from a central portion of the top surface while trending towards a side where the leaflet is located.

8. The medical device according to claim 7, wherein an included angle formed between a tangent plane of the central portion of the top surface and a tangent plane of an edge portion of the top surface ranges from 10° to 45°, and the edge portion of the top surface is close to the leaflet.

9. The medical device according to claim 6, wherein the valve allows blood to flow from a first chamber into a second chamber and prevents flowing in a reverse direction, the medical device further comprises a support member, the support member is configured to be located in the first chamber and be connected to the pad such that positioning the pad between the plurality of leaflets.

10. The medical device according to claim 9, wherein the support member has a main body portion and a pair of connecting portions, and the pair of connecting portions are configured to extend to two opposite sides of the pad along a width direction of the pad respectively and be connected to the pad.

11. The medical device according to claim 9, wherein the pad comprises an expandable mesh frame and a cover covering an outer side of the mesh frame, and the cover is disposed at a top end of the pad without covering any of the plurality of coaptation surfaces.

12. The medical device according to any one of claims 2 to 11, wherein the pad has a second coaptation surface, the second coaptation surface is configured to face the second leaflet and periodically coaptate and separate from the second leaflet as the second leaflet moves, and the second coaptation surface is configured to be concave in a direction away from the second leaflet.

13. The medical device according to claim 12, wherein the valve allows blood to flow from a first chamber into a second chamber and prevents flowing in a reverse direction, and the second coaptation surface is configured to extend gradually toward a side where the second leaflet is located while trending towards the first chamber.

14. The medical device according to claim 13, wherein the second coaptation surface has a first edge portion and a second edge portion located at two opposite ends of the second coaptation surface, the first edge portion is configured to be closer to the first chamber relative to the second edge portion, and an included angle formed between a tangent plane of the first edge portion and a tangent plane of the second edge portion ranges from greater than or equal to 10° to less than or equal to 45°.

15. The medical device according to claim 2, wherein the pad is configured to follow movement of the second leaflet, and the first coaptation surface is configured to periodically coaptate and separate from the first leaflet as the pad follows the movement of the second leaflet.

16. The medical device according to claim 15, wherein the valve allows blood to flow from a first chamber into a second chamber and prevents flowing in a reverse direction, the pad further has a top surface, the top surface is configured to face the first chamber and protrude toward the first chamber, and the top surface is configured to extend gradually toward a side where the first leaflet is located while trending towards the second chamber.

17. The medical device according to claim 16, wherein the top surface has a third edge portion and a fourth edge portion located at two opposite ends of the top surface, the fourth edge portion is configured to be closer to the second chamber relative to the third edge portion, and an included angle formed between a tangent plane of the third edge portion and a tangent plane of the fourth edge portion ranges from greater than or equal to 10° to less than or equal to 45°.

18. The medical device according to claim 15, wherein the pad also has a second coaptation surface, the second coaptation surface is configured to face the second leaflet and be concave in a direction away from the second leaflet, and remain coaptating the second leaflet as the pad follows the movement of the second leaflet.

19. The medical device according to claim 15, wherein the pad is configured to be attached to the second leaflet.

20. The medical device according to claim 15, wherein the valve allows blood to flow from a first chamber into a second chamber and prevent flowing in a reverse direction, the medical device further comprises a support member, the support member is configured to be located in a first chamber and position the pad between the first leaflet and the second leaflet, and the pad is connected to the support member in a manner that allows the pad to follow the movement of the second leaflet.

21. The medical device according to claim 1, wherein a thickness of the pad gradually increases while trending towards a first chamber.

22. The medical device according to claim 1, wherein the valve is a mitral valve, and a quantity of the plurality of leaflets is two; or, the valve is a tricuspid valve, and a quantity of the plurality of leaflets is three.
